# EUROPEAN PATENT SPECIFICATION

(11) **EP 2 948 088 B1**
(45) Date of publication and mention of the grant of the patent: **31.05.2017**
(21) Application number: 13713004.3
(22) Date of filing: 14.03.2013
(51) Int. Cl.: G09B 23/28, A61B 17/00, G09B 23/30

(54) **SURGICAL TRAINING SYSTEM**
CHIRURGISCHES TRAININGSSYSTEM
SYSTÈME DE FORMATION CHIRURGICALE

(30) Priority: 23.01.2013 US 201361755539 P
(43) Date of publication of application: 02.12.2015
(73) Proprietor: Boston Scientific Scimed, Inc., Maple Grove, MN 55311 (US)
(72) Inventor: WILL, Samuel, L., Minnetonka, MN 55343 (US)
(74) Representative: Vossius & Partner Patentanwälte Rechtsanwälte mbB
(86) International application number: PCT/US2013/031203
(87) International publication number: WO 2014/116278

(56) References cited:
- EP-A1- 2 110 799
- US-A1- 2005 084 833
- US-A1- 2009 263 775
- US-B1- 7 553 159

## Description

### BACKGROUND

Implantable electronic stimulator devices, such as neuromuscular stimulation devices, have been disclosed for use in the treatment of various pelvic conditions, such as urinary incontinence, fecal incontinence and sexual dysfunction. Such devices generally include one or more electrodes that are coupled to a control unit by electrode leads. Electrical signals are applied to the desired pelvic tissue of the patient through the electrode leads in order to treat the condition of the patient. The electrode leads are typically secured to the tissue using an anchor in the form of a helical coil. Exemplary implantable electronic stimulator devices and uses of the devices are disclosed in U.S. Pat. Nos. 6,354,991, 6,652,449, 6,712,772 and 6,862,480.

US2009263775 discloses a surgical simulation system used for training a physician during laparoscopic surgery, which provides a command to a robotic positioning assembly to adjust the position of the capture mechanism, based on the position of instruments relative to a cavity. US7553159 discloses a surgical training model for training surgeon to treat protocele in the abdominopelvic region, which has an elastomer mass encasing component model and a rigid skeletal frame with the same flexibility, density and consistency as that of the human body tissues.

One challenge with using a neuromuscular stimulation device to treat a pelvic condition is properly implanting the electrode lead at the targeted stimulation site. For example, urinary incontinence may be treated through electrical stimulation of the urinary sphincter.

One method of implanting the electrodes in the urinary sphincter involves delivering the electrodes into the urinary sphincter through a periurethral incision using an introducer. The physician generally positions the electrodes based on feel, but the physician may be aided by the use of imaging, such as X-ray, MRI, fluoroscopy, etc. Even with such imaging, multiple implantation attempts by the physician may be required before the electrodes are positioned properly. Additionally, with each implantation attempt, there is risk of urethra and bladder perforation.

To achieve clinical proficiency at performing electrode implantations, the clinician must practice the treatment. Such practice may involve the performance of an implantation procedure on a cadaver. Unfortunately, such practice opportunities are limited and time-consuming.

### SUMMARY

Embodiments of the invention are directed to a surgical training system and a surgical training method using the system. In some embodiments, the surgical training system includes an anatomical training model, three or more telemetry sensors attached to the training model, a training tool, a display, and a controller. The anatomical training model physically simulates human anatomical features. The training tool comprises at least one transmitter that is configured to emit a signal. The controller includes one or more processors that are configured to determine a location of the training tool relative to the training model using the sensors and the signal, and produce a virtual image of the training tool and anatomical features simulated by the training model on the display based on the location of the training tool.

In some embodiments, the training tool includes a gyroscope configured to output orientation information indicative of an orientation of the training tool relative to the training model. The controller is configured to determine an orientation of the training tool relative to the training model based on the orientation information, and produce the virtual image based on the orientation of the training tool.

In some embodiments, the system includes memory, a virtual tool stored in the memory, and a virtual model stored in the memory. The virtual tool defines a three-dimensional representation of the training tool within a tool coordinate system. The virtual model defines a three-dimensional representation of the training model within a model coordinate system. The virtual image includes the virtual tool and the virtual model, such as portions thereof, having relative positions and orientations that substantially match the relative positions and orientations of the training tool and the training model.

In some embodiments, the controller is configured to position the virtual tool within the virtual model based on the location of the training tool relative to the training model. In some embodiments, the controller is configured to position the virtual tool within the virtual model based on the orientation of the training tool relative to the training model.

In some embodiments, the training model physically simulates pelvic anatomy of a human. In some embodiments, the pelvic anatomy includes the vagina, the urethra, the bladder, the urinary sphincter, the anal canal, the anal sphincter, and/or pelvic bones.

In some embodiments, the training model includes one or more openings or passageways that simulate pelvic anatomy of a human. In some embodiments, the openings or passageways simulate the vagina, the urethra, and/or the anal canal of a human.

In some embodiments, the training tool comprises an introducer. In some embodiments, the training tool comprises a glove.

In some embodiments of the surgical training method, a training tool is positioned near an anatomical training model, which physically simulates human anatomical features. A position of the training tool relative to the training model is determined using a controller. An orientation of the training tool relative to the training model is determined using the controller. A virtual tool, which corresponds to the training tool, is located within a virtual model, which corresponds to the training model, based on the position and orientation of the training tool using the controller. The virtual tool and the virtual model are displayed on the display using the controller. In some embodiments, portions of the virtual tool and the virtual model are displayed on the display in the controller.

In some embodiments of a method, a position of the training tool relative to the training model is determined by emitting a signal from a transmitter attached to the tool, sensing the signal using three or more telemetry sensors attached to the training model, and determining the position of the training tool relative to the training model based on sensing the signal using the processor.

In some embodiments, the orientation of the training tool relative to the training model is determined by producing an output signal from a gyroscope attached to the tool, and determining the orientation of the training tool relative to the training model based on the output signal using the controller.

In some embodiments, the virtual tool is located within the virtual model by translating a tool coordinate system of the virtual tool to a model coordinate system of the virtual model using the controller.

In some embodiments, the training tool comprises an introducer having a distal end. In some embodiments, positioning the training tool relative to the anatomical training model comprises inserting the distal end of the introducer into a simulated urinary sphincter of the training model. In some embodiments of a method, the display of the virtual tool and the virtual model on the display comprises displaying the distal end and the urinary sphincter on the display.

In some embodiments of a method, the training tool is moved relative to the training model. A position of the training tool relative to the training model is determined using the controller. An orientation of the training tool relative to the training model is determined using the controller. The virtual tool is located within the virtual model based on the position and orientation of the training tool using the controller. The virtual tool and the virtual model are displayed on the display using the controller.

In some embodiments of the method, the training model physically simulates pelvic anatomy of a human. In some embodiments, the pelvic anatomy includes the vagina, the urethra, the bladder, the urinary sphincter, the anal canal, the anal sphincter, and/or pelvic bones.

This Summary is provided to introduce a selection of concepts in a simplified form that are further described below in the Detailed Description. This Summary is not indented to identify key features or essential features of the claimed subject matter, nor is it intended to be used as an aid in determining the scope of the claimed subject matter. The claimed subject matter is not limited to implementations that solve any or all disadvantages noted in the Background.

### BRIEF DESCRIPTION OF THE DRAWINGS

FIG. 1 is a simplified diagram of a surgical training system in accordance with embodiments of the invention.
FIGS. 2A-B respectively are simplified isometric and side views of a training model and a tool, in accordance with exemplary embodiments of the invention.
FIG. 3 is a simplified cross-sectional view of a tool and a training model in accordance with embodiments of the invention.
FIG. 4 is a simplified diagram illustrating an exemplary coordinate system of a virtual tool in accordance with embodiments of the invention.
FIGS. 5 and 6 are simplified diagrams illustrating exemplary coordinate systems of a virtual tool and a virtual model in accordance with embodiments of the invention.
FIG. 7 illustrates angular rotations of a tool coordinate system relative to model coordinate system.
FIG. 8 is a flowchart illustrating a surgical training method in accordance with embodiments of the invention.

### DETAILED DESCRIPTION OF ILLUSTRATIVE EMBODIMENTS

Embodiments of the invention are described more fully hereinafter with reference to the accompanying drawings. The various embodiments of the invention may, however, be embodied in many different forms and should not be construed as limited to the embodiments set forth herein. Rather, these embodiments are provided so that this disclosure will be thorough and complete, and will fully convey the scope of the invention to those skilled in the art. Elements that are identified using the same or similar reference characters refer to the same or similar elements.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

It will be understood that when an element is referred to as being "connected" or "coupled" to another element, it can be directly connected or coupled to the other element or intervening elements may be present. In contrast, if an element is referred to as being "directly connected" or "directly coupled" to another element, there are no intervening elements present.

It will be understood that, although the terms first, second, etc. may be used herein to describe various elements, these elements should not be limited by these terms. These terms are only used to distinguish one element from another. Thus, a first element could be termed a second element without departing from the teachings of the present invention.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

As will further be appreciated by one of skill in the art, the present invention may be embodied as methods, systems, and/or computer program products. Accordingly, the present invention may take the form of an entirely hardware embodiment, an entirely software embodiment or an embodiment combining software and hardware aspects. Furthermore, the present invention may take the form of a computer program product on a computer-usable storage medium having computer-usable program code embodied in the medium. Any suitable computer readable medium may be utilized including hard disks, CD-ROMs, optical storage devices, or magnetic storage devices.

The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM). Note that the computer-usable or computer-readable medium could even be paper or another suitable medium upon which the program is printed, as the program can be electronically captured, via, for instance, optical scanning of the paper or other medium, then compiled, interpreted, or otherwise processed in a suitable manner, if necessary, and then stored in a computer memory.

The invention is also described using flowchart illustrations and block diagrams. It will be understood that each block (of the flowcharts and block diagrams), and combinations of blocks, can be implemented by computer program instructions. These program instructions may be provided to a processor circuit, such as a microprocessor, microcontroller or other processor, such that the instructions which execute on the processor(s) create means for implementing the functions specified in the block or blocks. The computer program instructions may be executed by the processor(s) to cause a series of operational steps to be performed by the processor(s) to produce a computer implemented process such that the instructions which execute on the processor(s) provide steps for implementing the functions specified in the block or blocks.

Accordingly, the blocks support combinations of means for performing the specified functions, combinations of steps for performing the specified functions and program instruction means for performing the specified functions. It will also be understood that each block, and combinations of blocks, can be implemented by special purpose hardware-based systems which perform the specified functions or steps, or combinations of special purpose hardware and computer instructions.

Embodiments of the invention are directed to an electrode implantation training system 100 that allows a physician to practice an electrode implantation without the need for a cadaver. Additionally, the system 100 can provide feedback as to the performance of the physician.

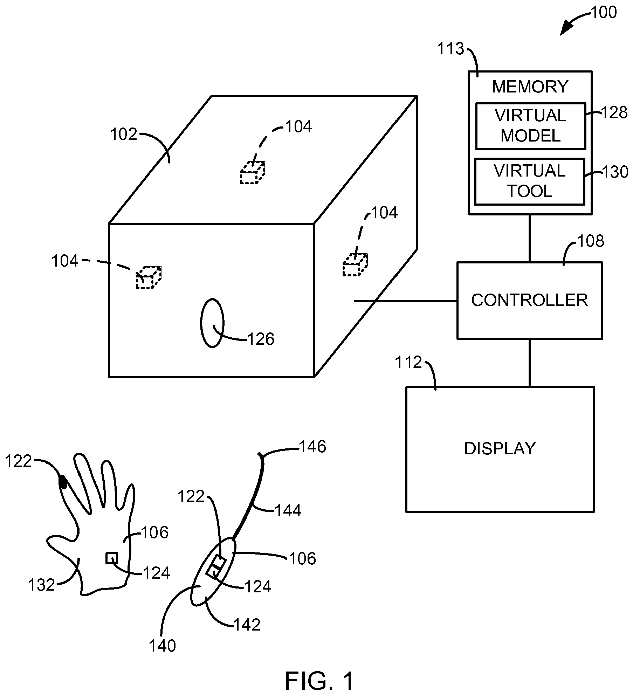

FIG. 1 is a simplified diagram of a surgical training system 100 in accordance with exemplary embodiments of the invention. In one embodiment, the system 100 includes a physical anatomical training model 102, telemetry sensors 104, one or more training tools 106, and a controller 108. In some embodiments, the system 100 includes a display 112. In some embodiments, the controller 108 represents one or more processors configured to execute program instructions contained in memory 113 or other location, to perform various functions described herein.

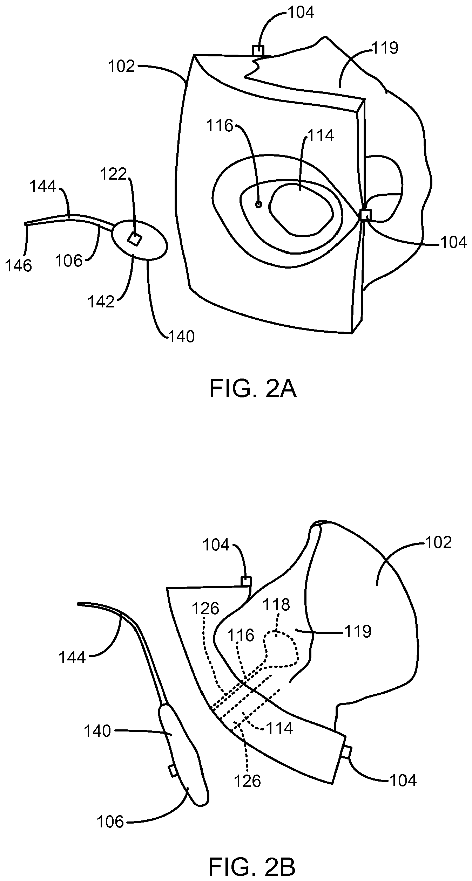

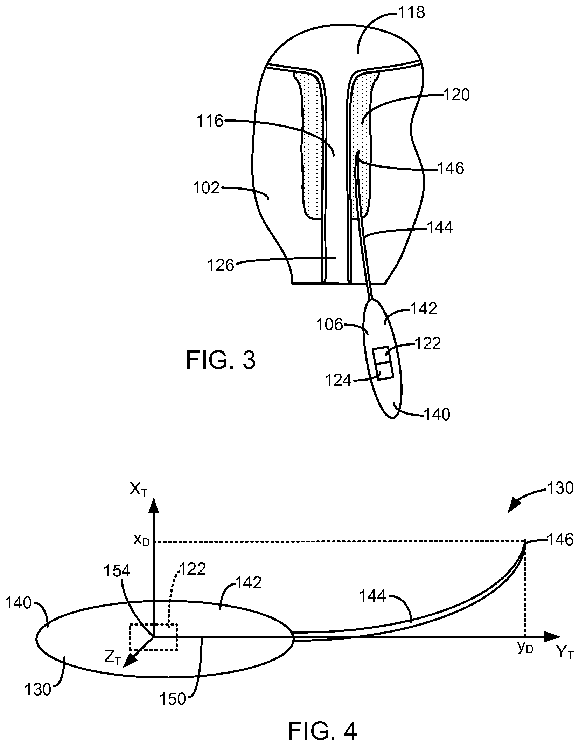

The training model 102 is generally configured to physically simulate the anatomy of a patient. In one embodiment, the model 102 simulates the pelvic anatomy of a patient, such as a female patient, as best illustrated in FIGS. 2A-B, which respectively show isometric, side, top and front views of a model 102, in accordance with exemplary embodiments of the invention. The model 102 preferably includes structures simulating anatomical features of a human patient, such as the vagina 114, the urethra 116, the urinary sphincter, the bladder 118, the anal canal, the anal sphincter, pelvic bones 119, and other anatomical features, some of which are illustrated in FIGS. 2A-B. FIG. 3 is a simplified cross-sectional view of a portion of the model 102 taken through the simulated urethra 116, the simulated urinary sphincter 120, and the simulated bladder 118 of the model 102, in accordance with embodiments of the invention.

In some embodiments, the training model 102 utilizes plastic, rubber, and other materials to simulate the shape and feel of anatomical features being physically modeled. In some embodiments, physical simulation of the anatomical features includes one or more openings or passageways 126 into the model 102, such as openings 126 for the simulated vagina 114 (FIG. 2B), the simulated urethra 116 (FIGS. 2B and 3), the simulated anal canal, and/or another simulated passageway of the model 102.

The tool 106 is used by the physician to perform a surgical training exercise on the training model 102, such as an electrode implantation training exercise on the training model 102. In some embodiments, the tool 106 is moved relative to the model 102.

Some embodiments of the system 100 provide virtual images of the tool 106 and the model 102 on the display 112 to provide feedback on the medical procedure being simulated. As the position and orientation of the tool 106 relative to the model 102 will change during the training session, it is necessary to determine the position of the tool 106 and orientation of the tool 106 relative to the model 102. In some embodiments, the tool 106 includes a transmitter 122 configured to emit a signal that is detected by the sensors 104. In some embodiments, the tool 106 includes a gyroscope 124 that outputs orientation information indicative of an orientation of the tool 106 relative to the model. The transmitter 106 and the gyroscope 124 may be conventional components.

In some embodiments, the system 100 includes a virtual model 128 stored in the memory 113 or other location, that defines a three-dimensional representation of anatomical features of the model 102 within a model coordinate system. Thus, when the training model simulates the vagina 114, the virtual model 128 includes a representation of the vagina 114. Additionally, the system 100 includes a virtual tool 130 stored in the memory 113 or other location, that defines a three-dimensional representation of the tool 106 within a tool coordinate system.

In some embodiments, the system 100 comprises at least three telemetry sensors 104, which may be conventional components. The sensors 104 are preferably displaced from each other around the periphery of the model 102. The signal emitted by the transmitter 122 is sensed by each of the sensors 104. The sensed signals are processed using the controller 108 to determine the distance the transmitter 122 is located from the sensors 104, and/or a point within the model coordinate system, using conventional triangulation techniques. In some embodiments, the controller 108 also processes orientation information from the gyroscope 124 to determine a relative orientation of the tool 106 within the model coordinate system.

In some embodiments, the controller 108 uses the position and orientation information for the tool 106 to produce a virtual image on the display 112 that includes the virtual tool 130, or a portion thereof, and the virtual model 128, or a portion thereof. In some embodiments, the virtual tool 130 is depicted in relatively the same position and orientation with respect to the virtual model 128, as the actual tool 106 is positioned and oriented with respect to the actual model 102. Exemplary virtual images produced on the display 112 may be similar to those provided in FIGS. 2A-B and FIG. 3, where the tool 106 represents the virtual tool 130, and the training model 102 represents the virtual model 128. Some elements of the actual tool 106 and the training model 102 may not be represented in the virtual tool 130 and the virtual model 128. For instance, the virtual tool 130 may not include the transmitter 122 or the gyroscope 124, and the virtual model 128 may not include the telemetry sensors 104, or other non-anatomical feature of the training model 102.

In some embodiments, the model 102 is used to simulate an electrode implantation to a targeted site within the pelvic region, such as a simulated anal sphincter, a simulated urinary sphincter 120 (FIG. 3), or other targeted site. Embodiments of the tool 106 include a glove 132, as shown in FIG. 1. In some embodiments, the transmitter 122 and/or gyroscope 124 is attached to the glove 132. In some embodiments, the glove 132 includes the transmitter 122 attached to a distal end of a finger section of the glove 132, such as the index finger section, as illustrated in FIG. 1. The controller 108 determines the location of the glove 132 relative to the training model 102 based on the sensed location of the glove 132 using the sensors 104. In some embodiments, the controller 108 determines an orientation of the glove 132 relative to the model 102 using the orientation information output from the gyroscope 124.

The glove 132 may be worn by the physician and used during the surgical training session, such as by inserting the finger containing the transmitter 122 through one of the openings 126 in the model 102 to palpate some of the anatomical features simulated by the model 102. For instance, the physician may palpate portions of the anatomical model 102 to guide an object to a desired simulated location within the model 102. As discussed above, a virtual tool 130 representing the glove 132, or at least the portion where the transmitter 122 is located, may be displayed on the display 112 along with the virtual model 128 using the controller 108.

In accordance with another embodiment, the tool 106 is in the form of an introducer or a mock introducer 140 (hereinafter "introducer"), which, as used herein, is a tool that is configured to deliver a distal end of an electrode lead to a targeted site in a patient. The introducer 140 may comprise a handle 142 and a needle or mock needle 144 (hereinafter "needle") having a distal tip 146. In some embodiments, the transmitter 122 and/or gyroscope 124 is contained on or within the handle 142, as shown in FIGS. 1 and 3. The transmitter 122 and/or gyroscope 108 may be supported in other locations on the introducer 140. As with the glove 132, the controller 108 may determine the location of the introducer 140 relative to the model 102 based on the sensed location of the introducer 140 using the sensors 104. In some embodiments, the controller 108 determines an orientation of the introducer 140 relative to the model 102 using the orientation information output from the gyroscope 124. In some embodiments, the controller 108 uses the position and orientation information for the introducer 140, the virtual tool 130 of the introducer 140, and the virtual model 128, to present a virtual image on the display 112 depicting virtual tool 130 and the virtual model 128, such as provided in FIGS. 2A-B and FIG. 3.

In some embodiments, the glove 132 and the introducer 140 may be used together during the training session. For instance, the physician may utilize the index finger of the glove 132, to which the transmitter 122 is attached, to palpate the simulated anatomy of the model 102 for the purpose of guiding the needle 144 to a desired targeted site. This may be useful when training to deploy an electrical lead to the targeted site, such as, for example, within a simulated urinary sphincter 120 of the model 102, as shown in FIG. 3. Here, virtual tools 130 of the glove 132 and the introducer 140 may be depicted on the display 112 relative to the virtual model 128, which may include a virtual image of the urinary sphincter 120, as shown in FIG. 3.

Techniques used to depict the tool 106 or features of the tool 106 relative to the model 102 on the display 112 are described below with reference to FIGS. 4-7. While the tool 106 is illustrated in the form of the introducer 140, the techniques are applicable to other forms of the tool 106, such as the glove 132, for example.

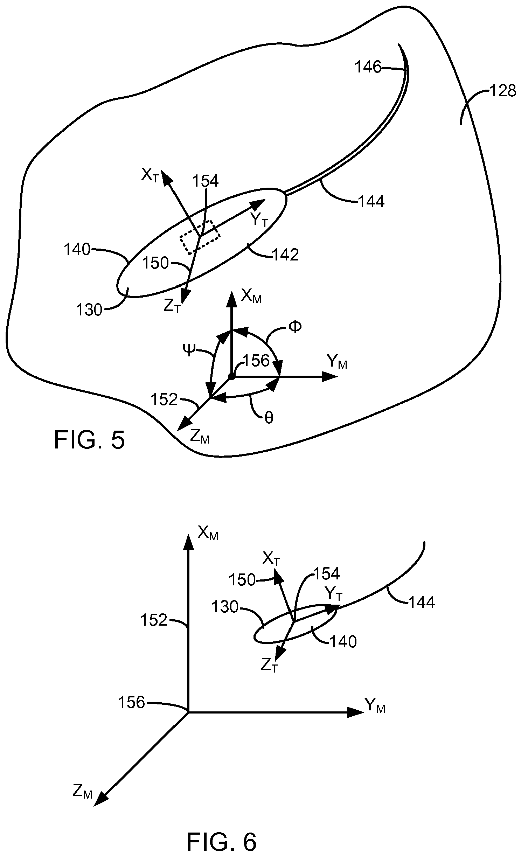

FIG. 4 is a simplified diagram illustrating an exemplary tool coordinate system 150 of the virtual tool 130, and FIGS. 5-6 are simplified diagrams illustrating the exemplary tool coordinate system 150 relative to an exemplary model coordinate system 152 of the virtual model 128, in accordance with embodiments of the invention. As mentioned above, the virtual tool 130 defines a three-dimensional representation of the tool 106 within the tool coordinate system 150, and the virtual model 128 defines a three-dimensional representation of the model 102 within the model coordinate system 152. References are made in the drawings to features of the corresponding tool 106 and training model 102 that may also be represented in the virtual tool 130 and the virtual model 128.

In some embodiments, the tool coordinate system 150 has axes X_{T}, Y_{T} and Z_{T} and an origin 154 that is set to the location of the transmitter 122, as shown in the simplified diagram provided in FIG. 4. The origin 154 may also be set to a location that is displaced from the location of the transmitter 122. The introducer 140 is defined based on coordinates within the tool coordinate system 150. For instance, if the distal end 146 of the introducer is aligned with the origin of the axis Z_{T}, the coordinates for the distal end 146 are x_{D}, y_{D}, 0, as indicated in FIG. 4. Other features of the introducer 140 are also mapped to the coordinate system 150 within the model 130.

In some embodiments, the model coordinate system 152 has axes X_{M}, Y_{M} and Z_{M} and an origin 156. The image or model 130 includes a mapping of features of the model within the coordinate system 152. In some embodiments, the origin 156 is set to the origin of the model 102 from which the controller 108 measures the relative position of the transmitter 122 using the sensors 104.

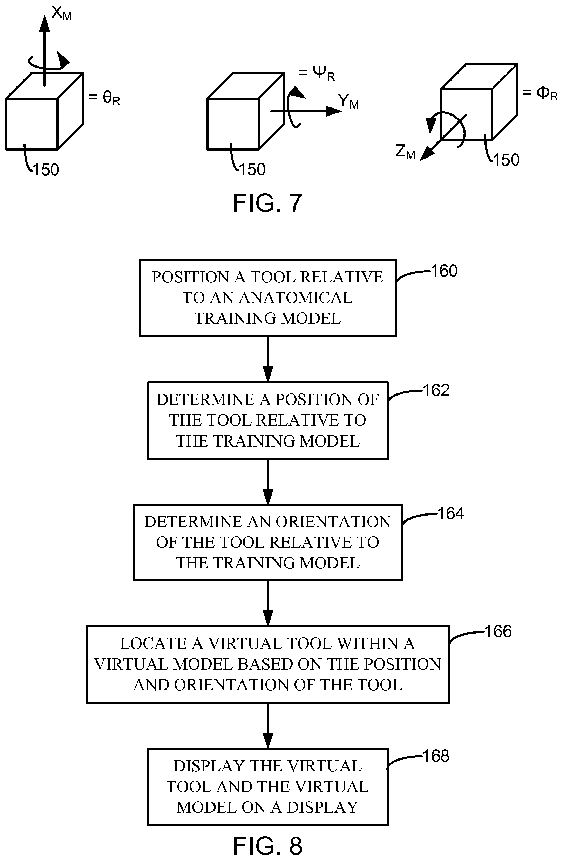

As mentioned above, the gyroscope 124 outputs an orientation of the tool 106 relative to the model 102. In some embodiments, the controller 108 translates this orientation information into an orientation of the tool coordinate system 150 of the virtual tool 130 relative to the model coordinate system 152 of the virtual model 128. In some embodiments, the controller 108 uses the output from the gyroscope 124 to determine angles θ, Ψ and Φ, at which the tool coordinate system 150 must be rotated to respectively align the axes X_{T}, Y_{T}, and Z_{T} of the tool coordinate system 150 with the axes X_{M}, Y_{M} and Z_{M} of the model coordinate system 152. For instance, the controller 108 uses the output from the gyroscope 124 to determine the angle θ_{R} that the tool coordinate system 150 must be rotated about the axis X_{M}, the angle Ψ_{R} that the tool coordinate system 150 must be rotated about the axis Y_{M}, and the angle Φ_{R} that the tool coordinate system 150 must be rotated about the axis Z_{M} to respectively align the axes X_{T}, Y_{T} and Z_{T} of the tool coordinate system 150 with the axes X_{M}, Y_{M} and Z_{M} of the model coordinate system 152, as illustrated in FIG. 7.

As mentioned above, the controller 108 determines the position of the transmitter 122 relative to the model 102, and preferably relative to a point within the model 102 corresponding to the origin 156 of the model coordinate system 152, using the outputs from the sensors 104 produced in response to sensing the emitted signal from the transmitter 122. This establishes a position of the origin of the tool coordinate system 150 relative to the model coordinate system 152 and, thus, a position of the virtual tool 130 within the virtual model 128. The orientation of the virtual tool 130 relative to the virtual model 128 is determined by the controller 108 based on the output from the gyroscope 124. The controller 108 can then generate a virtual image of the virtual tool 130, or a portion thereof, such as the distal end 146 of the introducer 140, within the virtual model 128 in a desired manner on the display 112.

In some embodiments, the calculation of the position and orientation of the virtual tool 130 relative to the virtual model 128, and the display of the virtual tool 130 and the virtual model 128, or portions thereof, on the display 112, is performed substantially in real time by the controller 108. This allows the system 100 to provide the handler of the tool 106 with real time feedback during the training session. Thus, as the tool 106 is moved relative to the model 102, the position and orientation information produced by the sensors 104 and the gyroscope 124 are sampled and processed by the controller 108 at a sufficiently high rate that the corresponding movement of the virtual tool 130 is provided on the display 112 in substantially real time. In other embodiments, snapshots of the current position of the virtual tool 130 may be provided on the display 112 as desired, such as at predetermined intervals, for example.

FIG. 8 is a flowchart illustrating a surgical training method using the system 100 in accordance with embodiments of the invention. At 160 of the method, a tool 106 is positioned near an anatomical training model 102, which physically simulates human anatomical features. At 162, a position of the tool 106 is determined relative to the training model 102 using a controller 108. An orientation of the tool 106 relative to the training model 102 is determined using the controller 108, at 164. At 166, a virtual tool 130 corresponding to the tool 106 is located within a virtual model 128 corresponding to the training model 102, based on the position and orientation of the tool 106 using the controller 108. At 168, the virtual tool 130 and the virtual model 128, or portions thereof, are produced on a display 112 using the controller 108. In some embodiments, the image of the virtual tool 130 on the display has a position and orientation relative to the virtual model 128 that substantially matches the position and orientation of the tool 106 relative to the training module 102.

Embodiments of the tool 106 include one or more embodiments of the glove 132 and/or the introducer 140 described above. The tool 106 may take on other forms depending on the surgical or medical procedure being simulated by the system 100.

Embodiments of the anatomical training model 102 include one or more embodiments of the training model 102 described above. In some embodiments, the training model 102 simulates pelvic anatomical features of the human, such as the vagina, the urethra, the urinary sphincter, the bladder, the anal canal, the anal sphincter, and pelvic bones, for example.

In some embodiments of step 162, a signal is emitted from a transmitter 122 attached to the tool 106, such as shown in FIG. 1. The signal is sent using three or more telemetry sensors 104 that are attached to the training model 102, as shown in FIG. 1. The position of the tool 106 relative to the training model 102 is determined based on the sensing of the emitted signal by the sensors 104 using the processor 108.

In some embodiments of step 164, an output signal is generated from a gyroscope 124 that is attached to the tool 106, as shown in FIG. 1. The output signal from the gyroscope is indicative of an orientation of the tool 106 relative to the model 102. The controller determines the orientation of the tool 106 relative to the training model 102 based on the output signal.

In some embodiments of step 166, a tool coordinate system 150 of the virtual tool 130 is translated to a model coordinate system 152 of the virtual model 128 using the controller 108. This may be accomplished in accordance with any of the embodiments described above. For instance, the tool coordinate system 150 may be rotated, as shown in FIG. 7, to place it in alignment with the model coordinate system 152 based on the orientation information output from the gyroscope 124. Additionally, the virtual tool 130 may be positioned within the virtual model 128 based on the position of the tool 106 relative to the model 102, as described above.

Some embodiments of the method relate to an electrode implantation within the pelvic region of the patient. For instance, the training may involve the simulated implantation of an electrode within the urinary sphincter 120 or anal sphincter simulated by the training model 102. In some embodiments, the tool 106 comprises an introducer 140 having a distal end 146. Some embodiments of step 160 of the method comprises inserting the distal end 146 of the introducer 140 into the simulated urinary sphincter 120 of the training model 102, as shown in FIG. 3. Some embodiments of step 166 include displaying the distal end 146 and the urinary sphincter 120 on the display 112.

In some embodiments of the method, the tool 106 is moved relative to the training model 102. The position and orientation of the tool relative to the training model 102 are determined using the controller 108. The virtual tool 130 is then located within the virtual model 128 based on the position and orientation of the tool 106 using the controller 108. The virtual tool 130 and the virtual model 128 are then displayed on the display 112 using the controller 108.

It is understood that the system 100 described herein may be configured to provide training for various surgical procedures by adjusting the anatomy simulated by the model 102. Additionally, the tool 106 utilized by the surgeon during the training may be adapted to the tools or objects used during the surgical procedure.

The invention is defined in the appended claims.

## Claims

1. A surgical training system (100) comprising:
an anatomical training model (102) physically simulating human anatomical features, wherein the training model physically simulates pelvic anatomy of a human selected from the group consisting of the vagina (114), the urethra (116), the bladder (118), the urinary sphincter (120), the anal canal, the anal sphincter, and pelvic bones (119);
three or more telemetry sensors (104) attached to the training model;
a training tool (106) comprising at least one transmitter (122) configured to emit a signal;
a display (112); and
a controller (108) comprising one or more processors configured to:
determine a location of the training tool relative to the training model using the sensors and the signal; and
produce a virtual image of the training tool and anatomical features simulated by the training model on the display based on the location of the training tool.

2. A system according to claim 1, wherein:
the training tool comprises a gyroscope (124) configured to output orientation information indicative of an orientation of the training tool relative to the training model; and
the controller is configured to determine an orientation of the training tool relative to the training model based on the orientation information, and produce the virtual image based on the orientation of the training tool.

3. A system according to any of claims 1-2, comprising:
memory (113);
a virtual tool (130) stored in the memory, the virtual tool defining a three-dimensional representation of the training tool within a tool coordinate system (150); and
a virtual model (128) stored in the memory, the virtual model defining a three-dimensional representation of the training model within a model coordinate system (152);
wherein the virtual image includes the virtual tool and the virtual model having relative positions and orientations that substantially match the relative positions and orientations of the training tool and the training model.

4. A system according to claim 3, wherein the controller is configured to position the virtual tool within the virtual model based on the location of the training tool relative to the training model.

5. A system according to any of claims 3-4, wherein the controller is configured to position the virtual tool within the virtual model based on the orientation of the training tool relative to the training model.

6. A system according to any of claims 1-5, wherein the training tool comprises an introducer (140).

7. A system according to any of claims 1-6, wherein the training tool comprises a glove (132).

8. A surgical training method comprising:
positioning (160) a training tool (106) near an anatomical training model (102), which physically simulates human anatomical features, wherein the training model physically simulates pelvic anatomy of a human selected from the group consisting of the vagina (114), the urethra (116), the bladder (118), the urinary sphincter (120), the anal canal, the anal sphincter, and pelvic bones (119);
determining (162) a position of the training tool relative to the training model using a controller (108);
determining (164) an orientation of the training tool relative to the training model using the controller;
locating (166) a virtual tool (130), which corresponds to the training tool, within a virtual model (128), which corresponds to the training model, based on the position and orientation of the training tool using the controller;
displaying (168) the virtual tool and the virtual model on a display (112) using the controller.

9. A method according to claim 8, wherein determining a position of the training tool relative to the training model comprises:
emitting a signal from a transmitter (122) attached to the tool;
sensing the signal using three or more telemetry sensors (104) attached to the training model; and
determining the position of the training tool relative to the training model based on sensing the signal using the processor.

10. A method according to any of claims 8-9, wherein determining an orientation of the training tool relative to the training model comprises:
producing an output signal from a gyroscope (124) attached to the tool; and
determining the orientation of the training tool relative to the training model based on the output signal using the controller.

11. A method according to any of claims 8-10, wherein locating a virtual tool within a virtual model comprises translating a tool coordinate system (150) of the virtual tool to a model coordinate system (152) of the virtual model using the controller.

12. A method according to any of claims 8-11, wherein:
the training tool comprises an introducer (140) having a distal end (146); and
positioning a training tool relative to an anatomical training model comprises inserting the distal end of the introducer into a simulated urinary sphincter (120) of the training model.

13. A method according to any of claims 8-12, wherein displaying the virtual tool and the virtual model on a display comprises displaying the distal end and the urinary sphincter on the display.

14. A method according to any of claims 8-13, further comprising:
moving the training tool relative to the training model;
determining a position of the training tool relative to the training model using the controller;
determining an orientation of the training tool relative to the training model using the controller;
locating the virtual tool within the virtual model based on the position and orientation of the training tool using the controller;
displaying the virtual tool and the virtual model on the display using the controller.

## Patentansprüche

1. Chirurgisches Trainingssystem (100), das aufweist:
ein anatomisches Trainingsmodell (102), das menschliche anatomische Merkmale körperlich simuliert, wobei das Trainingsmodell eine Beckenanatomie eines Menschen körperlich simuliert, die aus der Gruppe ausgewählt ist, die aus der Vagina (114), der Urethra (116), der Blase (118), dem Urethrasphinkter (120), dem Analkanal, dem Analsphinkter und Beckenknochen (119) besteht;
drei oder mehr Telemetriesensoren (104), die am Trainingsmodell angebracht sind;
ein Trainingswerkzeug (106) mit mindestens einem Sender (122), der so konfiguriert ist, dass er ein Signal abstrahlt;
ein Display (112); und
eine Steuerung (108) mit einem oder mehreren Prozessoren, die so konfiguriert sind, dass sie:
eine Lage des Trainingswerkzeugs relativ zum Trainingsmodell mit Hilfe der Sensoren und des Signals bestimmen; und
ein virtuelles Bild des Trainingswerkzeugs und der durch das Trainingsmodell simulierten anatomischen Merkmale auf dem Display auf der Grundlage der Lage des Trainingswerkzeugs erzeugen.

2. System nach Anspruch 1, wobei:
das Trainingswerkzeug ein Gyroskop (124) aufweist, das so konfiguriert ist, dass es Orientierungsinformationen als Angabe einer Orientierung des Trainingswerkzeugs relativ zum Trainingsmodell ausgibt; und
die Steuerung so konfiguriert ist, dass sie eine Orientierung des Trainingswerkzeugs relativ zum Trainingsmodell auf der Grundlage der Orientierungsinformationen bestimmt und das virtuelle Bild auf der Grundlage der Orientierung des Trainingswerkzeugs erzeugt.

3. System nach Anspruch 1 oder 2, das aufweist:
einen Speicher (113);
ein virtuelles Werkzeug (130), das im Speicher gespeichert ist, wobei das virtuelle Werkzeug eine dreidimensionale Darstellung des Trainingswerkzeugs in einem Werkzeugkoordinatensystem (150) definiert; und
ein virtuelles Modell (128), das im Speicher gespeichert ist, wobei das virtuelle Modell eine dreidimensionale Darstellung des Trainingsmodells in einem Modellkoordinatensystem (152) definiert;
wobei das virtuelle Bild das virtuelle Werkzeug und das virtuelle Modell mit Relativpositionen und Orientierungen aufweist, die im Wesentlichen den Relativpositionen und Orientierungen des Trainingswerkzeugs und des Trainingsmodells entsprechen.

4. System nach Anspruch 3, wobei die Steuerung so konfiguriert ist, dass sie das virtuelle Werkzeug im virtuellen Modell auf der Grundlage der Lage des Trainingswerkzeugs relativ zum Trainingsmodell positioniert.

5. System nach Anspruch 3 oder 4, wobei die Steuerung so konfiguriert ist, dass sie das virtuelle Werkzeug im virtuellen Modell auf der Grundlage der Orientierung des Trainingswerkzeugs relativ zum Trainingsmodell positioniert.

6. System nach einem der Ansprüche 1 bis 5, wobei das Trainingswerkzeug ein Einführinstrument (140) aufweist.

7. System nach einem der Ansprüche 1 bis 6, wobei das Trainingswerkzeug einen Handschuh (132) aufweist.

8. Chirurgisches Trainingsverfahren, das aufweist:
Positionieren (160) eines Trainingswerkzeugs (106) nahe einem anatomischen Trainingsmodell (102), das menschliche anatomische Merkmale körperlich simuliert, wobei das Trainingsmodell eine Beckenanatomie eines Menschen körperlich simuliert, die aus der Gruppe ausgewählt ist, die aus der Vagina (114), der Urethra (116), der Blase (118), dem Urethrasphinkter (120), dem Analkanal, dem Analsphinkter und Beckenknochen (119) besteht;
Bestimmen (162) einer Position des Trainingswerkzeugs relativ zum Trainingsmodell mit Hilfe einer Steuerung (108);
Bestimmen (164) einer Orientierung des Trainingswerkzeugs relativ zum Trainingsmodell mit Hilfe der Steuerung;
Lokalisieren (166) eines virtuellen Werkzeugs (130), das dem Trainingswerkzeug entspricht, in einem virtuellen Modell (128), das dem Trainingsmodell entspricht, auf der Grundlage der Position und Orientierung des Trainingswerkzeugs mit Hilfe der Steuerung;
Anzeigen (168) des virtuellen Werkzeugs und des virtuellen Modells auf einem Display (112) mit Hilfe der Steuerung.

9. Verfahren nach Anspruch 8, wobei das Bestimmen einer Position des Trainingswerkzeugs relativ zum Trainingsmodell aufweist:
Abstrahlen eines Signals von einem Sender (122), der am Werkzeug angebracht ist;
Erfassen des Signals mit Hilfe von drei oder mehr Telemetriesensoren (104), die am Trainingsmodell angebracht sind; und
Bestimmen der Position des Trainingswerkzeugs relativ zum Trainingsmodell auf der Grundlage der Erfassung des Signals mit Hilfe des Prozessors.

10. Verfahren nach Anspruch 8 oder 9, wobei das Bestimmen einer Orientierung des Trainingswerkzeugs relativ zum Trainingsmodell aufweist:
Erzeugen eines Ausgangssignals von einem Gyroskop (124), das am Werkzeug angebracht ist; und
Bestimmen der Orientierung des Trainingswerkzeugs relativ zum Trainingsmodell auf der Grundlage des Ausgangssignals mit Hilfe der Steuerung.

11. Verfahren nach einem der Ansprüche 8 bis 10, wobei das Lokalisieren eines virtuellen Werkzeugs in einem virtuellen Modell aufweist: Übersetzen eines Werkzeugkoordinatensystems (150) des virtuellen Werkzeugs in ein Modellkoordinatensystem (152) des virtuellen Modells mit Hilfe der Steuerung.

12. Verfahren nach einem der Ansprüche 8 bis 11, wobei:
das Trainingswerkzeug ein Einführinstrument (140) mit einem distalen Ende (146) aufweist; und
das Positionieren eines Trainingswerkzeugs relativ zu einem anatomischen Trainingsmodell aufweist:
Einführen des distalen Endes des Einführinstruments in einen simulierten Urethrasphinkter (120) des Trainingsmodells.

13. Verfahren nach einem der Ansprüche 8 bis 12, wobei das Anzeigen des virtuellen Werkzeugs und des virtuellen Modells auf einem Display aufweist: Anzeigen des distalen Endes und des Urethrasphinkters auf dem Display.

14. Verfahren nach einem der Ansprüche 8 bis 13, das ferner aufweist:
Bewegen des Trainingswerkzeugs relativ zum Trainingsmodell;
Bestimmen einer Position des Trainingswerkzeugs relativ zum Trainingsmodell mit Hilfe der Steuerung;
Bestimmen einer Orientierung des Trainingswerkzeugs relativ zum Trainingsmodell mit Hilfe der Steuerung;
Lokalisieren des virtuellen Werkzeugs im virtuellen Modell auf der Grundlage der Position und Orientierung des Trainingswerkzeugs mit Hilfe der Steuerung;
Anzeigen des virtuellen Werkzeugs und des virtuellen Modells auf dem Display mit Hilfe der Steuerung.

## Revendications

1. Système d'apprentissage chirurgical (100) comprenant :
un modèle d'apprentissage anatomique (102) simulant physiquement des caractéristiques anatomiques humaines, dans lequel le modèle d'apprentissage simule physiquement une anatomie pelvienne d'un humain sélectionné dans le groupe consistant en le vagin (114), l'urètre (116), la vessie (118), le sphincter urinaire (120), le canal anal, le sphincter anal, et les os pelviens (119) ;
trois capteurs de télémétrie (104) ou plus attachés au modèle d'apprentissage ;
un outil d'apprentissage (106) comprenant au moins un émetteur (122) configuré pour émettre un signal ;
un afficheur (112) ; et
un contrôleur (108) comprenant un ou plusieurs processeurs configurés pour :
déterminer un emplacement de l'outil d'apprentissage par rapport au modèle d'apprentissage en utilisant les capteurs et le signal ; et
produire une image virtuelle de l'outil d'apprentissage et des caractéristiques anatomiques simulées par le modèle d'apprentissage sur l'afficheur sur la base de l'emplacement de l'outil d'apprentissage.

2. Système selon la revendication 1, dans lequel :
l'outil d'apprentissage comprend un gyroscope (124) configuré pour délivrer des informations d'orientation indicatives d'une orientation de l'outil d'apprentissage par rapport au modèle d'apprentissage ; et
le contrôleur est configuré pour déterminer une orientation de l'outil d'apprentissage par rapport au modèle d'apprentissage sur la base des informations d'orientation, et pour produire l'image virtuelle sur la base de l'orientation de l'outil d'apprentissage.

3. Système selon l'une quelconque des revendications 1 et 2, comprenant :
une mémoire (113) ;
un outil virtuel (130) mémorisé dans la mémoire, l'outil virtuel définissant une représentation tridimensionnelle de l'outil d'apprentissage dans un système de coordonnées d'outil (150) ; et
un modèle virtuel (128) mémorisé dans la mémoire, le modèle virtuel définissant une représentation tridimensionnelle du modèle d'apprentissage dans un système de coordonnées de modèle (152) ;
dans lequel l'image virtuelle comprend l'outil virtuel et le modèle virtuel ayant des positions et des orientations relatives qui correspondent sensiblement aux positions et aux orientations relatives de l'outil d'apprentissage et du modèle d'apprentissage.

4. Système selon la revendication 3, dans lequel le contrôleur est configuré pour positionner l'outil virtuel dans le modèle virtuel sur la base de l'emplacement de l'outil d'apprentissage par rapport au modèle d'apprentissage.

5. Système selon l'une quelconque des revendications 3 et 4, dans lequel le contrôleur est configuré pour positionner l'outil virtuel dans le modèle virtuel sur la base de l'orientation de l'outil d'apprentissage par rapport au modèle d'apprentissage.

6. Système selon l'une quelconque des revendications 1 à 5, dans lequel l'outil d'apprentissage comprend un dispositif d'introduction (140).

7. Système selon l'une quelconque des revendications 1 à 6, dans lequel l'outil d'apprentissage comprend un gant (132).

8. Procédé d'apprentissage chirurgical comprenant :
le positionnement (160) d'un outil d'apprentissage (106) à proximité d'un modèle d'apprentissage anatomique (102), qui simule physiquement des caractéristiques anatomiques humaines, dans lequel le modèle d'apprentissage simule physiquement une anatomie pelvienne d'un humain sélectionnée dans le groupe consistant en le vagin (114), l'urètre (116), la vessie (118), le sphincter urinaire (120), le canal anal, le sphincter anal, et les os pelviens (119) ;
la détermination (162) d'une position de l'outil d'apprentissage par rapport au modèle d'apprentissage en utilisant un contrôleur (108) ;
la détermination (164) d'une orientation de l'outil d'apprentissage par rapport au modèle d'apprentissage en utilisant le contrôleur ;
le placement (166) d'un outil virtuel (130), qui correspond à l'outil d'apprentissage, dans un modèle virtuel (128), qui correspond au modèle d'apprentissage, sur la base de la position et de l'orientation de l'outil d'apprentissage en utilisant le contrôleur ;
l'affichage (168) de l'outil virtuel et du modèle virtuel sur un afficheur (112) en utilisant le contrôleur.

9. Procédé selon la revendication 8, dans lequel la détermination d'une position de l'outil d'apprentissage par rapport au modèle d'apprentissage comprend :
l'émission d'un signal à partir d'un émetteur (122) attaché à l'outil ;
la détection du signal en utilisant trois capteurs de télémétrie (104) ou plus attachés au modèle d'apprentissage ; et
la détermination de la position de l'outil d'apprentissage par rapport au modèle d'apprentissage sur la base de la détection du signal en utilisant le processeur.

10. Procédé selon l'une quelconque des revendications 8 et 9, dans lequel la détermination d'une orientation de l'outil d'apprentissage par rapport au modèle d'apprentissage comprend :
la production d'un signal de sortie à partir d'un gyroscope (124) attaché à l'outil ; et
la détermination de l'orientation de l'outil d'apprentissage par rapport au modèle d'apprentissage sur la base du signal de sortie en utilisant le contrôleur.

11. Procédé selon l'une quelconque des revendications 8 à 10, dans lequel le placement d'un outil virtuel dans un modèle virtuel comprend la translation d'un système de coordonnées d'outil (150) de l'outil virtuel vers un système de coordonnées de modèle (152) du modèle virtuel en utilisant le contrôleur.

12. Procédé selon l'une quelconque des revendications 8 à 11, dans lequel :
l'outil d'apprentissage comprend un dispositif d'introduction (140) comportant une extrémité distale (146) ; et
le positionnement d'un outil d'apprentissage par rapport à un modèle d'apprentissage anatomique comprend l'insertion de l'extrémité distale du dispositif d'introduction dans un sphincter urinaire simulé (120) du modèle d'apprentissage.

13. Procédé selon l'une quelconque des revendications 8 à 12, dans lequel l'affichage de l'outil virtuel et du modèle virtuel sur un afficheur comprend l'affichage de l'extrémité distale et du sphincter urinaire sur l'afficheur.

14. Procédé selon l'une quelconque des revendications 8 à 13, comprenant en outre :
le déplacement de l'outil d'apprentissage par rapport au modèle d'apprentissage ;
la détermination d'une position de l'outil d'apprentissage par rapport au modèle d'apprentissage en utilisant le contrôleur ;
la détermination d'une orientation de l'outil d'apprentissage par rapport au modèle d'apprentissage en utilisant le contrôleur ;
le placement de l'outil virtuel dans le modèle virtuel sur la base de la position et de l'orientation de l'outil d'apprentissage en utilisant le contrôleur ;
l'affichage de l'outil virtuel et du modèle virtuel sur l'afficheur en utilisant le contrôleur.
